# EUROPEAN PATENT SPECIFICATION

(11) **EP 0 990 026 B1**
(45) Date of publication and mention of the grant of the patent: **12.05.2004**
(21) Application number: 98934912.1
(22) Date of filing: 04.06.1998
(51) Int. Cl.: C12N 9/00

(54) **HIGH-ACTIVITY PHYTASE GRANULATE**
HOCHAKTIVE PHYTASEN ENTHALTENDE GRANULAT
COMPOSITIONS DE PHYTASE A HAUTE ACTIVITE

(30) Priority: 04.06.1997 EP 97201641; 04.06.1997 US 48611 P
(43) Date of publication of application: 05.04.2000
(60) Divisional application: 03077926.8
(73) Proprietor: BASF Aktiengesellschaft, 67056 Ludwigshafen (DE)
(72) Inventor: BARENDSE, Rudolf, Carolus, Maria, NL-2613 CM Delft (NL); MEESTERS, Gabriel, Marinus, Henricus, NL-2614 TJ Delft (NL); ANDELA, Carl, Sidonius, Maria, NL-2625 LL Delft (NL)
(74) Representative: Maurer, Stefanie
(86) International application number: PCT/EP1998/003328
(87) International publication number: WO 1998/055599

(56) References cited:
- EP-A- 0 257 996
- EP-A- 0 304 332
- EP-A- 0 501 375
- EP-A- 0 758 018
- WO-A-94/03072
- GB-A- 2 225 784
- US-A- 4 740 469
- STROUCKEN W P J ET AL: "EXTRUDING VS PELLETING OF A FEED MIXTURE LOWERS APPARENT NITROGEN DIGESTIBILITY IN DOGS" JOURNAL OF THE SCIENCE OF FOOD AND AGRICULTURE, vol. 71, no. 4, 1 August 1996, pages 520-522, XP000595883

## Description

### Field of the Invention

The present invention relates to the preparation and formulation of phytase enzymes and their use to prepare granulates for feed-enzymes in animal feeds.

### Background of the Invention

The use of various enzymes such as phytases in animal, e.g. livestock, feed is becoming more common. These enzymes are included in order to improve nutrient or mineral uptake from the feed by the animal, and may also help digestibility. They are usually produced by culturing microorganisms in large scale fermenters operated by industrial enzyme producers. At the end of the fermentation the resulting "broth" is usually subjected to a series of filtration steps to separate the biomass (the microorganisms) from the desired enzyme (in solution). The enzyme solution is either then sold as a liquid (often after addition of various stabilizers) or processed to a dry formulation.

Enzyme liquid and dry formulations are used on a commercial scale by the animal feed industry. Liquid formulations may be added to the feed after pelleting in order to avoid heat inactivation of the enzyme which would occur during the pelleting process.

Dry formulations usually involve steam pelleting where the feed is subjected to steam injection(s) prior to pelleting. In the subsequent pelleting step the feed is forced through a matrix or die and the resulting strips are cut into suitable pellets of variable length. During this process temperatures may rise to 60-95°C.

Phytases are enzymes that (at least partially) hydrolyse phytate (*myo*-inositolhexakis phosphate) to myo-inositol and inorganic phosphate. These enzymes are found in wheat bran, plant seeds, animal intestines and can be produced by microorganisms. Phytases are provided in animal feeds because, as they are able to degrade phytase, they can increase the availability of phosphorus and other nutritional components to the animal. Phytases can also increase the digestibility of calcium.

Phosphorus is an essential element for the growth of organisms. For livestock, the feed is often supplemented with inorganic phosphorus in order to obtain good growth in monogastric animals. There is however often no need for this in feedstuffs of ruminants because microorganisms present in the rumen produce enzymes that catalyse the conversion of phytase to inositol and inorganic phosphate. The degration of phytate is often desirable because phytic.acid can be anti-nutritional as it chelates useful minerals such as calcium, zinc, magnesium and iron; and can also react adversely with proteins thereby decreasing their bioavailability to the animal. The addition of phytase may also reduce the amount of inorganic feed that needs to be added, and so less phosphorus is excreted in the manure which is better for the environment.

The gene for various phytase enzymes have been cloned and expressed. EP-A-0,420,358 (Gist-Brocades) describes the expression of microbial phytases.

In a later application EP-A-0,684,313 (Hoffmann-La Roche) describes a DNA sequences coding for various polynucleotides having phytase activity.

EP-A-0,758,018 (Gist-Brocades) refers to methods of improving the stability of enzymes, especially for use as animal feeds, and refers to phytases.

WO-A-94/03612 (Alko) describes the production of phytase degrading enzymes in *Trichoderma* while WO-A-97/16076 (Novo Nordisk) describes enzyme-containing preparations for use in the manufacture of animal feeds comprising various hydrophobic substances.

Animal feed represents one of the largest costs incurred in keeping livestock and other animals. Furthermore, additives such as enzymes like phytase can add significantly to the cost of animal feed. One aim of the present invention is to be able to provide phytase compositions that are cheaper to produce. This can be achieved by being able to manufacture high activity or highly concentrated phytase compositions. There are several factors that have allowed the present Applicant to be able to make these high activity compositions, and these will be discussed later.

An additional advantage in being able to make high activity phytase compositions that has been noticed by the Applicant is that these compositions can show a marked increase in stability, especially during a pelleting process in the preparation of animal feed (pellets), and so are more likely to retain the higher phytase activity than the compositions of the prior art over time.

### Description of the Invention

In a first aspect of the present invention there is provided a process for the preparation of an aqueous liquid comprising a phytase, the process comprising:
(a) culturing in an aqueous medium a microorganism of the genus *Aspergillus* or *Trichoderma* having a heterologous phytase gene under the control of a glucoamylase (for *Aspergillus*) or cellobiohydrolase (for *Trichoderma*) promoter, under conditions that allow recombinant expression of the phytase, where the medium comprises, as a feed for the microorganism, an assimilable carbon source and an assimilable nitrogen source;
(b) filtering the aqueous medium to remove the microorganisms to give an aqueous filtrate; and
(c) subjecting the filtrate from (b) to ultrafiltration to give an aqueous liquid having a phytase concentration of at least 14,000 FTU/g.

This process has been found to provide a particularly high concentration of phytase in the resulting aqueous composition. This has allowed the preparation of other phytase compositions, also at high activity levels, which means that not only is the process cheaper (per unit of enzyme activity), but also the more concentrated phytase-containing compositions have been found to be much more stable than their less concentrated counterparts.

The microorganisms are preferably of the species *Aspergillus niger, Aspergillus oryzae* or *Trichoderma reesei*. For the *Aspergillus* organisms, the phytase gene is suitably under the control of a glucoamylase (or amyloglucosidase, AG) promoter. For Trichoderma organisms, it is preferred to use a cellobiohydrolase promoter.

The assimilable carbon source can comprise glucose and/or maltodextrin, and/or the assimilable nitrogen source consists of ammonium ions. The glucose and ammonium ions can be the only assimilable carbon or nitrogen sources in the aqueous medium. That is to say, it is contemplated that no complex carbon or nitrogen sources are used. The ammonium ions may be provided either as ammonia or an ammonium salt. Preferred ammonium salts include ammonium nitrate, ammonium sulphate and ammonium phosphate.

Preferably the carbon and/or nitrogen source is supplied to the culture medium during the fermentation process. The rate of supply of either source can be substantially the same as it is consumed by the microorganisms. Thus the carbon and/or nitrogen source can be provided in a continuous or continual manner. The carbon and nitrogen sources can be provided separately, or in the same supply.

The resulting aqueous liquid can have a phytase concentration of at least 16,000, and possibly even 18,000 or more, FTU/g.

By using these particular organisms under these conditions the filtrate will be relatively concentrated. This allows it to be subjected to ultra-filtration. In some prior art methods, the resulting filtrate contains too much debris and other substances to allow ultra-filtration (the filter clogs). However, in the process of the present invention, the filtrate is relatively "clean" which allows the filtrate to be subjected to ultra-filtration, without any further processing, and by ultra-filtration a particularly high concentrated aqueous composition can therefore be obtained.

Prior art methods have discussed the possibility of subjecting either the filtrate or the aqueous composition to either crystallization and/or colour removal steps, for example charcoal filtration. However, both of these additional steps (which would add to the cost of producing the phytase) can be dispensed with in the present invention.

Preferably the microorganisms do not possess, or at least do not express, a glucoamylase gene. This means that the microorganism can devote more energy to the production of the phytase.

The microorganism may possess multiple copies of the phytase gene. This has been found to increase the production levels of the phytase because there are more phytase genes to be expressed.

The aqueous composition may be substantially free of taka-amylase.

In the process of the invention it is preferred that (substantially) all of the carbon and/or nitrogen sources have been consumed by the microorganisms before the filtering in (b) takes place. This can be achieved by allowing fermentation to continue for some time after the last supply of carbon and/or nitrogen sources have taken place. Alternatively, one can allow the fermentation to continue beyond the stage when all of the carbon and/or nitrogen sources have been added. The advantage of this, as will be apparent, is that the aqueous composition can then be (substantially) free of the carbon and/or nitrogen sources (e.g. the glucose and/or ammonium ions). Once again, this can make for a cleaner aqueous liquid, which may contain fewer by-products. By reducing the number of by-products one can minimise the number of processing steps required to be able to either use the aqueous liquid, or to be able to obtain a desired high phytase activity.

The most preferred organism is *Aspergillus* niger. Also preferred is that the phytase is expressed in a microorganism with a glucoamylase signal sequence.

The resulting aqueous phytase-containing liquid can then be used for a variety of purposes, although its application in animal feeds is specifically contemplated here. A second aspect of the invention relates to this aqueous liquid, such as preparable by a process of the first aspect, comprising phytase at a concentration of at least 14,000 FTU/g.

In the specification a "phytase" means not only naturally occurring phytase enzymes, but any enzyme that possesses phytase activity, for example the ability to catalyse the reaction involving the removal or liberation of inorganic phosphorous (phosphate) from *myo*-inositol phosphates. Preferably the phytase will belong to the class EC 3.1.3.8. The phytase itself is preferably a fungal phytase, such as derived from an *Aspergillus* or *Trichoderma* species.

The invention can also provide processes for the preparation of phytase formulations in the form of granulates that use an edible carbohydrate polymer as a carrier. The carrier may be in particulate or powder form. The phytase-containing aqueous liquid, such as a solution or a slurry, can be mixed with the solid carrier and allowed to absorb onto the carrier. During or after the mixing, the phytase-containing liquid and the carrier are processed into a granulate, which can then subsequently be dried. The use of the carbohydrate carrier may allow the absorption of large amounts of the composition (and therefore phytase). The mixture may be used to form a plastic paste or non-elastic dough that can readily be processed into granules, for example it is extrudable. Suitably the carrier is non-fibrous which allows for easier granulation: fibrous materials can prevent granulation by extrusion.

A number of prior art documents that refer to pellets containing various enzymes, but these find use as detergents, often in washing compositions. In contrast, the present application finds use in animal feeds and for that reason the granulates of the invention are edible (by animals) and preferably also digestible. It will therefore not be surprising that the granulates, granules and compositions of the invention are free of soap, detergents and bleach or bleaching compounds, zeolites, binders, fillers (TiO₂, kaolin, silicates, talc etc) to name but a few.

The edible carbohydrate polymer should be chosen so that it is edible by the animal for whom the feed is intended, and preferably digestible as well. The polymer preferably comprises glucose (e.g. a glucose-containing polymer), or (C₆H₁₀O₅)ₙ, units. Preferably the carbohydrate polymer comprises α-D-glucopyranose units, amylose (a linear (1→4) α-D-glucan polymer) and/or amylopectin (a branched D-glucan with α-D-(1→4) and α-D-(1→6) linkages). Starch is the preferred carbohydrate polymer. Other suitable glucose-containing polymers that can be used instead of, or in addition to starch, include α-glucans, β-glucans, pectin (such as proto-pectin), and glycogen. Derivatives of these carbohydrate polymers, such as ethers and/or esters, thereof are also contemplated, although gelatinised starch is often avoided. Suitably the carbohydrate polymer is water-insoluble.

In the examples described herein corn-, potato- and rice-starch is used. However, starch obtained from other (e.g. plant, such as vegetable or crop) sources such as tapioca, cassava, wheat, maize, sago, rye, oat, barley, yam, sorghum, or arrowroot is equally applicable. Similarly both native or modified (e.g. dextrin) types of starch can be used in the invention. Preferably the carbohydrate (e.g. starch) contains little or no protein, e.g. less than 5% (w/w), such as less than 2% (w/w) preferably less than 1% (w/w).

At least 15% (w/w) of the solid carrier may comprise the carbohydrate polymer (such as starch). Preferably, however, at least 30% (w/w) of the solid carrier comprises the carbohydrate, optimally at least 40% (w/w). Advantageously the major component of the solid carrier is the carbohydrate (e.g. starch), for example more than 50% (w/w), preferably at least 60% (w/w), suitably at least 70% (w/w), and optimally at least 80% (w/w). These weight percentages are based on the total weight of the non-enzymatic components in the final dry granulate.

The amount of phytase-containing liquid that can be absorbed onto the carrier is usually limited by the amount of water that can be absorbed. For natural, granular, starch this can vary between 25 - 30% (w/w), without using elevated temperatures (that cause the starch to swell). In practice the percentage of enzyme liquid to be added to the carbohydrate will often be much larger than this because the enzyme containing liquid will usually contain a significant amount of solids. The phytase solution can contain about 25% (w/w) solids, as a result of which the carbohydrate (e.g. starch) and phytase solution can be mixed at a ratio of carbohydrate:phytase solution of 0.5:1 to 2:1, e.g. 1.2:1 to 1.6:1, such as at a ratio of about 60% (w/w):40% (w/w), respectively. Preferably the amount of liquid added to the solid carrier is such that (substantially) all the water in the (aqueous) liquid is absorbed by the carbohydrate present in the solid carrier.

At elevated temperatures starch and other carbohydrate polymers can absorb much larger amounts of water under swelling. For this reason the carbohydrate polymer is desirably able to absorb water (or enzyme-containing aqueous liquids). For example, corn starch can absorb up to three times its weight of water at 60°C and up to ten times at 70°C. The use of higher temperatures in order to absorb a greater_amount enzyme-containing liquid is thus contemplated by the present invention, and indeed is preferable especially when dealing with thermostable phytase enzymes. For these enzymes therefore the mixing of the solid carrier and liquid can be conducted at elevated temperatures (e.g. above ambient temperature), such as above 30°C, preferably above 40°C and optimally above 50°C. Alternatively or in addition the liquid may be provided at this temperature.

However, in general, non-swelling conditions at lower (e.g. ambient) temperatures are preferred to minimise activity loss arising from instability of (heat sensitive) phytases at higher temperatures. Suitably the temperature during the mixing of the enzyme and the liquid is from 20 to 25°C.

The mechanical processing used in the present invention for making the mixture of the phytase-containing liquid and the solid carrier into granules (in other words granulating) can employ known techniques frequently used in food, feed and enzyme formulation processes. This may comprise expansion, extrusion, spheronisation, pelleting, high shear granulation, drum granulation, fluid bed agglomeration or a combination thereof. These processes are usually characterised by an input of mechanical energy, such as the drive of a screw, the rotation of a mixing mechanism, the pressure of a rolling mechanism of a pelleting apparatus, the movement of particles by a rotating bottom plate of a fluid bed agglomerator or the movement of the particles by a gas stream, or a combination thereof. These processes allow the solid carrier (e.g. in the form of a powder), to be mixed with the phytase-containing liquid (an aqueous solution or slurry), and so subsequently granulated.

In yet a further embodiment of the invention the granulate (e.g. an agglomerate) is formed by spraying or coating the phytase-containing liquid onto the carrier, such as in a fluid bed agglomerator. Here the resulting granules can include an agglomerate as can be produced in a fluid bed agglomerator.

Preferably the mixing of the phytase-containing liquid and the solid carrier additionally comprises kneading of the mixture. This may improve the plasticity of the mixture in order to facilitate granulation (e.g. extrusion).

If the granulate is formed by extrusion this is preferably performed at low pressure. This may offer the advantage that the temperature of the mixture being extruded will not, or only slightly, increase. Low-pressure extrusion includes extrusion for example in a Fuji Paudal basket- or dome- extruder. Preferably extrusion does not result in the temperature of the material being extruded to rise above 40°C. The extrusion may naturally produce granules (the granules may break off after passage through a die) or a cutter may be employed.

Suitably the granules will have a water content of from 30 to 40%, such as from 33 to 37%. The enzyme content is sutiably from 3 to 10%, e.g. from 5 to 9%.

The granules obtained can be subjected to rounding off (e.g spheronisation) such as in a spheromiser, e.g. a MARUMERISER™ machine and/or compaction. The granules can be spheronised prior to drying since this may reduce dust formation in the final granulate and/or may facilitate any coating of the granulate.

The granules can then be dried, such as in a fluid bed drier or, in case of the fluid bed agglomeration, can be immediately dried (in the agglomerator) to obtain (solid dry) granulates. Other known methods for drying granules in the food, feed or enzyme industry can be used by the skilled person. Suitably the granulate is flowable.

The drying preferably takes place at a temperature of from 25 to 60°C, such as 30 to 50°C. Here the drying may last from 10 minutes to several hours, such as from 15 to 30 minutes. The length of time required will of course depend on the amount of granules to be dried, but as a guide this is from 1 to 2 seconds per kg of granules.

After drying the granules, the resulting granulate preferably has a water content of from 3 to 10%, such as from 5 to 9%.

A coating may be applied to the granulate to give additional (e.g. favoured) characteristics or properties, like low dust content, colour, protection of the enzyme from the surrounding environment, different enzyme activities in one granulate or a combination thereof. The granules can be coated with a fat, wax, polymer, salt, unguent and/or ointment or a coating (e.g. liquid) containing a (second) enzyme or a combination thereof. It will be apparent that if desired several layers of (different) coatings can be applied. To apply the coating(s) onto the granulates a number of known methods are available which include the use of a fluidised bed, a high shear granulator, a mixer granulator, or a Nauta-mixer.

In other embodiments additional ingredients can be incorporated into the granulate e.g. as processing aids, for further improvement of the pelleting stability and/or the storage stability of the granulate. A number of such preferred additives are discussed below.

Salts may be included in the granulate, (e.g. with the solid carrier or liquid). Preferably (as suggested in EP-A-0,758,018) inorganic salt(s) can be added, which may improve the processing and storage stability of the dry phytase preparation. Preferred inorganic salts comprise a divalent cation, such as zinc, magnesium, and calcium. Sulphate is the most favoured anion.

Preferably (as suggested in EP-A-0,758,018) inorganic salt(s) can be added, which may improve the processing and storage stability of the dry enzyme preparation. Preferred inorganic salts are water soluble. They may comprise a divalent cation, such as zinc (in particular), magnesium, and calcium. Sulphate is the most favoured anion although other anions resulting in water solubility can be used. The salts may be added (e.g. to the mixture) in solid form. However, the salt(s) can be dissolved in the water or enzyme-containing liquid prior to mixing with the solid carrier. Suitably the salt is provided at an amount that is at least 15% (w/w based on the enzyme), such as at least 30%. However, it can be as high as at least 60% or even 70% (again, w/w based on the enzyme). These amounts can apply either to the granules or to the granulate. The granulate may therefore comprise less than 12% (w/w) of the salt, for example from 2.5 to 7.5%, e.g. from 4 to 6%.

If the salt is provided in the water then it can be in an amount of from 5 to 30% (w/w), such as 15 to 25%.

Further improvement of the pelleting stability may be obtained by the incorporation of hydrophobic, gel-forming or slow dissolving (e.g. in water) compounds. These may be provided at from 1 to 10%, such as 2 to 8%, and preferably from 4 to 6% by weight (based on the weight of water and solid carrier ingredients). Suitable substances include derivatised celluloses, such as HPMC (hydroxy-propyl-methyl-cellulose), CMC (carboxy-methyl-cellulose), HEC (hydroxy-ethyl-cellulose); polyvinyl alcohols (PVA); and/or edible oils. Edible oils, such as soy oil or canola oil, can be added (e.g. to the mixture to be granulated) as a processing aid, although as a rule hydrophobic substances (e.g. palm oil) are preferably absent.

Preferably the granules have a relatively narrow size distribution (e.g. they are monodisperse). This can facilitate a homogeneous distribution of the phytase in the granules and/or the enzyme granulate in the animal feed. The process of the invention tends to produce granulates with a narrow size distribution. However, if necessary, an additional step can be included in the process to further narrow the size distribution of the granules, such as screening. The size distribution of the granulate is suitably between 100 µm and 2000 µm, preferably between 200 µm and 1800 µm and optimally between 300 µm and 1600 µm. The granules may be of irregular (but preferably regular) shape, for example approximately spherical.

Other suitable enzyme(s) can be included in the animal feed which includes pet food. The function of these enzymes is often to improve the feed conversion rate, e.g. by reducing the viscosity or by reducing the anti-nutritional effect of certain feed compounds. Feed enzymes can also be used, such as to reduce the amount of compounds which are harmful to the environment in the manure. Preferred enzymes for these purposes are: carbohydrases, such as amylolytic enzymes and plant cell wall degrading enzymes of which include cellulases such as β-glucanases, hemicelluloses such as xylanases, or galactanases; peptidases, galactosidases, pectinases, esterases; proteases, preferably with a neutral and/or acidic pH optimum; and lipases, preferably phospholipases such as the mammalian pancreatic phospholipases A2. Preferably, the enzyme does not include starch degrading enzymes (for example amylases). In some embodiments proteases may be excluded as these may cause harm if ingested. If the enzyme is a plant cell wall degrading enzyme, for example a cellulase, and in particular a hemicellulose such as xylanase, then the final granulate may have an activity of the enzyme ranging from 3,000 to 100,000, preferably 5,000 to 80,000, and optimally 8,000 to 70,000, EXU/g. If the enzyme is a cellulase, such as β-gluconase, then the final granulate can have an enzyme activity of from 500 to 15,000, preferably from 1,000 to 10,000, and optimally from 1,500 to 7,000, BGU/g.

The granules may comprise from 5 to 20, e.g. from 7 to 15% of the enzyme(s). The enzyme may be naturally occurring or recombinant.

A preferred process according to the invention therefore comprises:
a. mixing the aqueous phytase-containing liquid and solid carrier comprising at least 15% (w/w) or an edible carbohydrate polymer, for example mixing the solid carrier with an aqueous enzyme-containing liquid;
b. optionally kneading the resulting mixture;
c. granulating, for example by mechanical processing, the mixture in order to obtain enzyme-containing granules, for example by using a granulator or by extrusion;
d. optionally spheronising the granules;
e. drying the resultant granules to obtain an enzyme-containing granulate.

During the entire process one will aim to keep the maximum temperature to which the enzyme(s) are exposed to below 80°C.

The granulates of the invention are suitable for use in the preparation of an animal feed. At its broadest this aspect of the invention covers a granulate comprising a phytase and an edible carbohydraft polymer, the granulate having an activity of at least 6,000 FTU/g.In such processes the granulates are mixed with feed substances, either as such, or as part of a premix. The characteristics of the granulates according to the invention allows their use as a component of a mixture which is well suited as an animal feed, especially if the mixture is steam treated and subsequently pelleted. The dried granules may be visible or distinguishable in such pellets.

Thus a third aspect of the present invention relates to a process for the preparation of animal feed, or a premix or precursor to an animal feed, the process comprising mixing a composition of the second aspect with one or more animal feed substances (e.g. seeds) or ingredients. This can then be sterilised, e.g. subjected to heat treatment. The resulting composition is then suitably processed into pellets.

A fourth aspect of the invention relates to a composition comprising a granulate of the second aspect, which is preferably an edible feed composition such as an animal feed. This composition is preferably in the form of pellets (there may be 1-5, e.g. 2 to 4, dried granules per pellet).

Suitably the composition comprises from 0.05 to 2.0, such as 0.3 to 1.0, optimally 0.4 to 0.6 FTU/g of the phytase. A xylanase may be present at from 0.5 to 50, e.g. 1 to 40 EXU/g. Alternatively or in addition a cellulase may be present at from 09.1 to 1.0, e.g. 0.2 to 0.4 BGU/g.

The composition can have a water content of from 10 to 20%, e.g. from 12-15%. The amount of enzyme(s) is suitably from 0.0005 to 0.0012%, such as at least 5ppm.

A fifth aspect relates to a process for promoting the growth of an animal, the process comprising feeding an animal with a diet that comprises a composition of the second aspect or a composition of the fourth aspect. Here, the animal diet can include either the granulate itself, or the granulate present in a feed.

A sixth aspect of the present invention relates to the use of compositions in, or as a component of, an animal feed or for use in an animal diet.

A seventh aspect of the present invention also relates to the use of a composition comprising at least 15% (w/w) of an edible carbohydrate polymer as a carrier for a phytase to improve the pelleting stability of the phytase.

Suitable animals include farm animals (pigs, poultry, livestock), non-ruminants or monogastric animals (pigs, fowl, poultry, marine animals such as fish), ruminants (bovine or ovine, e.g. cows, sheep, goats, deer, calves, lambs). Poultry includes chickens, hens and turkeys.

Preferred features and characteristics of one aspect of the invention are equally applicable to another *mutatis mutandis*.

The following Examples are presented merely to illustrate the invention, and are not intended, or to be construed as, being limiting.

### EXAMPLES

### EXAMPLE 1

### Fermentation of A. niger CBS 513.88

*Aspergillus niger* fungal spore preparations were made following standard techniques.

Spores and subsequently cells were transferred through a series of batch fermentations in Erlenmeyer flasks to a 101 fermenter. After growth in batch culture, the contents of this fermenter were used as inoculum for a final 500 litre batch fermentation.

The media used contains: 91g/l corn starch (BDH chemicals); ammonium 38g/l glucose.H₂O; 0.6g/l MgSO₄.7H₂O; 0.6g/l KCl; 0.2g/l FeSO₄.7H₂O and 12g/l KNO₃. The pH was maintained at 4.6 ± 0.3 by automatic titration with either 4N NaOH or 4N H₂SO₄.

Cells were grown at 28°C at an automatically controlled dissolved oxygen concentration at 25% air saturation. Phytase production reached a maximum level of 5-10 U/ml after 10 days of fermentation.

The fermentation was repeated using ammonium sulphate in place of corn starch (to give an equivalent assimilable nitrogen content).

### EXAMPLE 2

### Purification and characterization of phytase: phytase activity assay

100µl of broth filtrate (diluted when necessary) or supernatant or 100µl of demiwater as reference are added to an incubation mixture having the following composition:
- 0.25M sodium acetate buffer pH 5.5, or
- glycine HCl-buffer; pH 2.5
- 1mM phytic acid, sodium salt
- demiwater up to 900µl

The resulting mixture was incubated for 30 minutes at 37°C. The reaction was stopped by the addition of 1 ml of 10% TCA (trichloroacetic acid). After the reaction had terminated, 2 ml of reagent (3.66g of FeSO₄.7H₂O in 50ml of ammonium molybdate solution (2.5g (NH₄)₆Mo₇O₂₄.4H₂O and 8ml of H₂SO₄, diluted up to 250ml with demiwater)) was added.

The intensity of the blue colour was measured spectro-photometrically at 750nm. The measurements are indicative of the quantity of phosphate released in relation to a calibration curve of phosphate in the range of 0-1 mMol/l.

### EXAMPLE 3

### A. Phytase expression in A. niger CBS 513.86 transformed with expression vectors containing the A. Ficuum phytase gene fused to the promoter and/or signal sequences of the A. niger amyloglucosidase (AG) gene

To obtain overexpression of phytase in *A. niger* an expression cassette was derived in which the *A. ficuum* phytase gene was under control of the *A. niger* amyloglucosidase (AG) promoter in combination a signal sequence. For the longer leader sequence the AG promoter sequence was fused to the phytase encoding sequence including the phytase leader sequence which was fused to the phytase gene fragment encoding the mature protein (see for reference Example 10 of EP-A-0,420,358).

### B. Expression of the phytase gene under the control of the AG promoter in A. niger

The *A. niger* strain CBS 513.88 (deposited 10 October 1988) was transformed with 10µg DNA fragment by known procedures (e.g. see Example 9 of EP-A-0,420,358). Single *A. niger* transformants from each expression cassette were isolated, and spores were streaked on selective acetamide-agar plates. Spores of each transformant were collected from cells grown for 3 days at 37°C on 0.4% potato-dextrose (Oxoid, England) agar plates. Phytase production was tested in shake flasks under the following conditions:

Approximately 1 x 10⁸ spores were inoculated in 100ml pre-culture medium containing (per litre): 1g KH₂PO₄; 30g maltose; 5g yeast extract; 10g casein-hydrolysate; 0.5g MgSO₄.7H₂O and 3g Tween 80. The pH was adjusted to 5.5.

After growing overnight at 34°C in a rotary shaker, 1ml of the growing culture was inoculated in a 100ml main-culture containing (per litre): 2g KH₂PO₄; 70g maltodextrin (maldex MDO₃ , Amylum); 12.5g yeast extract; 25g casein-hydrolysate; 2g K₂SO₄; 5g MgSO₄.7H₂O; 0.03g ZnCl₂; 0.02g CaCl₂; 0.05 MnSO₄.4H₂O and FeSO₄. The pH was adjusted to 5.6.

The mycelium was grown for at least 140 hours. Phytase production was measured as described in Example 2. glucose and ammonium sulphate as the carbon and nitrogen sources. The broth was filtered to give a filtrate which was separated from the biomass. Using the expression cassette PFYT3 (AG-promoter/phytase leader) a maximum phytase activity of 280 U/ml was obtained.

### EXAMPLE 4

### Purification of Phytase from filtrate

The purification to obtain highly purified phytase was as follows:
1. Cation exchange chromatography at pH 4.9
2. Cation exchange chromatography at pH 3.8
3. Anion exchange chromatography at pH 6.3
4. Ultrafiltration

1. The phytase filtrate was diluted 20 times with water and the pH was set at 4.9. This material was passed through a S Sepharose Fast Flow column equilibrated with a 20mM citric acid/NaOH pH 4.9 buffer. The unbound material, with phytase, was collected and used for the next step.
2. The pH 4.9 material was brought to pH 3.8 and the phytase was bound on a S Sepharose Fast Flow column equilibrated with 2-mM citric acid/NaOH pH 3.8 buffer. The phytase was eluted from the column with a 20mM NaPO₄, 50mM NaCl pH 7.6 buffer.
3. The pooled phytase fractions from the second cation exchange step were pH adjusted to 6.3 and the phytase was bound onto a Q Sepharose Fast Flow column equilibrated with a 10mM KPO₄ pH 6.3 buffer. The phytase was eluted using a gradient to 1M NaCl in the same buffer.

| Summary Purification Results | |
|---|---|
| Sample | Purification Factor |
| Starting Filtrate | 1 |
| After Cation exchanger pH 4.9 | 1.07 |
| After Cation exchanger pH 3.8 | 1.2 |
| After Anion exchanger | 1.46 |

The final (anion exchanged) product containing 10mg protein/ml was concentrated ten-fold by ultrafiltration using an Amicon Stirred Cell (2L module) with a Kalle E35 membrane at 3 bar.

The final concentration for purified phytase reached 280-300g/l (28-30%). With a specific activity of 100 FTU/mg protein, this results in a phytase activity of 28,000-30,000 FTU/g.

### EXAMPLE 5

### High Activity Phytase Stability Tests

To demonstrate that a higher enzyme concentration (in granules made using the high activity phytase liquid) gives a higher pelleting stability, granulates with an increasing enzyme concentration were made and the pelleting stability of these samples were tested.

### Comparative Sample A: Preparation of a corn starch-based low active enzyme granulate by mixing, kneading, extrusion, spheronisation and drying.

A mixture was prepared by mixing and kneading 73% (w/w) corn starch and low concentration 4% (w/w) phytase Ultra Filtrate and 23% (w/w) water. This mixture was extruded using a Nica E-220 basket extruder to obtain a wet extrudate which was spheronised in a Fuji Paudal Marumeriser™ for 2 minutes to obtain round particles of an average diameter of 600µm. These particles were subsequently dried in a Glatt GPCG 1.1 fluid bed dryer. The final activity of the granulate was 610 FTU/g.

### Comparative Sample B: Preparation of a corn starch-based middle active enzyme granulate by mixing, kneading, extrusion, spheronisation and drying.

A mixture was prepared by mixing and kneading 70% (w/w) corn starch and 17% (w/w) phytase Ultra Filtrate and 13% (w/w) water. This mixture was extruded using a Nica E-220 basket extruder to obtain a wet extrudate which was spheronised in a Fuji Paudal Marumeriser for 2 minutes to obtain round particles of an average diameter of 600µm. These particles were subsequently dried in a Glatt GPCG 1.1 fluid bed dryer. The final activity of the granulate was 4170 FTU/g.

### Sample C

Preparation of a corn starch-based high active enzyme granulate by mixing, kneading, extrusion, spheronisation and drying.

A mixture was prepared by mixing and kneading 67% (w/w) corn starch and 30% (w/w) of the phytase Ultra Filtrate prepared in Example 4 (but diluted to 18,400 FTU/g) and 3% (w/w) water. This mixture was extruded using a Nica E-220 basket extruder to obtain a wet extrudate which was spheronised in a Fuji Paudal Marumeriser for 2 minutes to obtain round particles of an average diameter of 600µm. These particles were subsequently dried in a Glatt GPCG 1.1 fluid bed dryer. The final activity of the granulate was 6830 FTU/g.

### Comparison of the pelleting stabilities

The different enzyme granulates were subsequently placed in a pelleting trial and their pelleting stability compared. The pelleting trial consists of mixing the enzyme granulates with a feed premix at respectively 1500, 320 and 200ppm. These mixtures were pre-treated by steam injection to give a temperature rise to 75°C, after which the mixtures were pelleted in a pelleting machine to obtain the feed pellets at a temperature of 82°C, which were subsequently dried. This type of process is typical for the feed industry to obtain feed pellets.

Table 1 summarises the results of the pelleting trials. It is apparent that the two granules with the highest enzyme concentration had much higher pelleting stability.

**TABLE 1**

| Results of the pelleting tests | | | | |
|---|---|---|---|---|
| Sample number | Granulate activity in FTU/g | Temp. of meal (°C) | Temp. pellets (°C) | Enzyme yield after pelleting (%) |
| Comp.(A) | 610 | 75 | 82 | <17 |
| Comp.(B) | 4,170 | 75 | 82 | 37 |
| (C) | 6,830 | 75 | 82 | 48 |

### EXAMPLE 6

### Preparation of a potato starch-based enzyme granulate containing soy oil and MgSO₄ additions by mixing, kneading, pelleting and drying

In a mixer/kneader 30 kg of potato starch was added and 2.5 kg of Soy oil was mixed in. Subsequently a phytase ultra-filtrate derived from *Aspergillus* (16,840 FTU/g) was added containing MgSO₄.7H₂O (3.5 kg of MgSO₄.7H₂O was dissolved in 14 kg of ultra-filtrate). The product was mixed thoroughly in the kneader, then extruded and dried in a fluid bed drier as in Example 1. This resulted in a product of 5870 FTU/g.

### EXAMPLE 7

### Preparation of a rice starch-based enzyme granulate by mixing, kneading, extrusion, spheronisation and drying

A mixture was prepared by mixing and kneading 62% (w/w) rice starch and 38% (w/w) of the same phytase ultra-filtrate used in Example 6. This mixture was extruded using the Fuji Paudal basket extruder to obtain a wet extrudate which was then spheronised in the MARUMERISER™ for one minute to obtain round particles of an average diameter of 785 µm. These particles were subsequently dried in a fluid bed drier as in Example 1. The final activity of the granulate was 7280 FTU/g.

### EXAMPLE 8

### Preparation of a corn starch-based enzyme granulate containing an HPMC addition by mixing, kneading, extrusion, spheronisation and drying

An enzyme preparation was obtained by kneading a mixture of 54% (w/w) of corn starch, 5% of HPMC (hydroxy-propyl-methyl-cellulose) and 41% (w/w) of the phytase ultra-filtrate used in Example 6. This mixture was extruded using the Fuji Paudal basket extruder to obtain a wet extrudate which was spheronised in the MARUMERISER™ for one minute to obtain round particles of an average diameter of 780 µm. These were subsequently dried in a fluid bed drier for 20 minutes at 40°C bed temperature, and 75°C inlet temperature. The thus obtained dry enzyme granulate had an activity of 8470 FTU/g.

### EXAMPLE 9

### Preparation of a corn starch-based enzyme granulate containing an HEC addition by mixing, kneading, extrusion, spheronisation and drying

An enzyme preparation was obtained by mixing and kneading 54% (w/w) of corn starch, 5% (w/w) of HEC (hydroxy-ethyl-cellulose) with 41% (w/w) of the same phytase ultra-filtrate used in Example 6. This mixture was extruded using the Fuji Paudal basket extruder to obtain a wet extrudate which was spheronised in the MARUMERISER™ for one minute to obtain round particles of an average diameter of 780 µm. These were subsequently dried in a fluid bed drier for 20 minutes at 40°C bed temperature, and 75°C inlet temperature. The thus obtained dry enzyme granulates had an activity of 8410 FTU/g.

### EXAMPLE 10

An ultrafiltrate of 18,000 FTU/g was employed, derived from the ultrafiltrate from Example 4, and diluted.

### Samples

The activity of the 3 samples prepared were 610(A, Comparative); 4170 (B,Comparative) and 6830 (C) FTU/g. This gave three feeds of activity 1.153,.1.685 and 1.745 FTU/g feed, respectively.

The first sample, 150g was mixed with 20 kg feed as described below. After this the premix was mixed with 80kg feed and divided in two parts to become feed for two trials at two different temperatures. The second sample was 153.6g in 20kg feed. This 20,153.6g sample was divided in two equal portions of 10.076kg. Each portion was then mixed with 230kg feed to get the meal for the tests.

For the third trial 96g of granulate was mixed with 20kg feed and divided in two portions of 10.048g. Each portion was then mixed with 230kg feed to get the meal for the tests. The pelleting speed was 600kg/h.

The feed mixture consisted of:

| | |
|---|---|
| Corn | 20.00 % |
| Wheat | 30.00 % |
| Soybeans (heated) | 10.00 % |
| Soy (coarse meal 46.7/3.7) | 18.20 % |
| Tapioca (65 % starch) | 6.97 % |
| Animal meal (56.5/10.9) | 4.00 % |
| Fish meal (70.6 % re) | 1.00 % |
| Feather meal, hydr. | 1.00 % |
| Soy oil/ maize oil | 1.30 % |
| Animal fat | 4.00 % |
| Vit./Min.premix ( maize) | 1.00 % |
| Calcium carbonate | 0.85 % |
| Mono-calcium phosphate | 1.05 % |
| Salt | 0.26 % |
| L-Lysine HCl | 0.16 % |
| DL-Methionine | 0.21 % |

The three mixtures were then pelleted. The feed was fed into a conditioner where direct steam was added to the meal. The temperature rose to 75°C. Subsequently the meal exited the pelletiser where it was pushed through a die plate with 5 mm holes and 65 mm thick. The temperature of the feed at this point rose another 4°C to 79°C.

The activity of the three feeds was 10.11(A); 10.04(B) and 9.81(C).

The results of this test for residual activity were: 63(A); 66(B) and 72%(C) respectively for the original 610; 4170; 6830 and FTU/g samples. This shows that even with similar activities (B and C) the highest activity formulation (C; 6830 FTU/g, of the invention) gave a much higher pelleting stability. This was 6% higher than for (comparative) Sample B, remarkable as only a 3% increase was observed (from A to B) with a very large increase in activity (610 to 4170 FTU/g).

## Claims

1. A process for the preparation of an aqueous liquid comprising a phytase, the process comprising:
(a) culturing in an aqueous medium a microorganism of the genus *Aspergillus* or *Trichoderma* having a heterologous phytase gene under the control of a glucoamylase (for *Aspergillus*) or cellobiohydrolase (for *Trichoderma*) promoter, under conditions that allow recombinant expression of the phytase, where the medium comprises, as a feed for the microorganism an assimilable carbon source and an assimilable nitrogen source consisting of ammonium ions;
(b) filtering the aqueous medium to remove the microorganisms to give an aqueous filtrate; and
(c) subjecting the filtrate from (b) to ultrafiltration to give an aqueous liquid, having a phytase concentration of at least 14,000 FTU/g.

2. A process according to claim 1 where the microorganism is *Aspergillus niger, Aspergillus oryzae* or *Trichoderma reesei*.

3. A process according to claim 1 or 2 wherein the microorganism does not possess, or does not express, a glucoamylase (AG) gene and/or the microorganism possesses multiple copies of the phytase gene and/or the phytase is expressed in the microorganism with a glucoamylase signal sequence.

4. A process according to any preceding claim wherein the aqueous liquid is substantially free of taka-amylase and/or wherein substantially all of the carbon and nitrogen sources in the medium have been consumed by the microorganisms before filtering in (b).

5. A process according to any preceding claim wherein the aqueous liquid is free of the carbon and/or nitrogen sources.

6. A process according to any preceding claim wherein neither the aqueous filtrate or the aqueous liquid are subjected to crystallisation and/or a colour-removal step.

7. A process according to any preceding claim wherein the resulting aqueous liquid has a phytase activity of 18,000 FTU/g or more.

8. An aqueous liquid preparable by a process according to any preceding claim comprising a phytase at a concentration of at least 14,000 PTU/g.

9. An aqueous liquid according to claim 8 which is derived from a culture medium in which the phytase was expressed.

10. A process for the preparation of a phytase-containing granulate, suitable for use in an animal feed, the process comprising processing a solid carrier comprising at least 15% (w/w) of an edible carbohydrate polymer and an aqueous liquid according to claim 8 or 9 to obtain phytase-containing granules.

11. A process according to claim 10 wherein the process comprises mixing the aqueous liquid and carrier, and kneading the resulting mixture and optionally subsequently drying the granules.

12. A process according to claim 10 or 11 wherein the process comprises:
a) mixing the aqueous liquid containing the phytase with the solid carrier;
b) mechanically processing the mixture obtained in a) to obtain enzyme-containing granules; and
c) drying the enzyme-containing granules obtained in b).

13. A process according to claim 12 wherein the processing comprises extrusion, pelleting, high-shear granulation, expansion, fluid bed agglomeration or a combination thereof.

14. A phytase-containlng granulate preparable by a process according to any of claims 10 to 13.

15. A granulate comprising dried granules formed from a phytase and a solid carrier which comprises at least 15% (w/w) of an edible carbohydrate polymer.

16. A granulate according to claim 15 wherein the granules comprise at least one divalent cation and/or one or more hydrophobic, gel-forming or water insoluble compound(s).

17. A granulate according to claim 16 wherein the hydrophobic, gel-forming or water insoluble compound comprises a derivatised cellulose, polyvinyl alcohol (PVA) or an edible oil.

18. A granulate according to claim 17 wherein the derivatised cellulose is hydroxy-propyl-methyl-cellulose, carboxy-methyl-cellulose or hydroxy-ethyl-cellulose and/or the edible oil is soy oil or canola oil.

19. A granulate according to any one of claims 15 to 18 which additionally comprises an endo-xylanase and/or β-glucanase and/or wherein the carrier comprises starch.

20. A granulate according to any of claims 15 to 19 wherein the phytase is other than a heat tolerant (thermostable) phytase and/or a fungal phytase, the said fungal phytase optionally derived from an *Aspergillus* or *Trichoderma* species.

21. A process for the preparation of an animal feed, a premix or precursor to the animal feed, the process comprising mixing a phytase-containing granulate as defined in any of claims 15 to 20 with one or more animal feed substance(s) or ingredient(s).

22. A process according to claim 21 wherein the mixture of feed substance(s) and composition or granulate is treated with steam, pelletised and optionally dried.

23. A composition comprising a granulate according to any of claims 15 to 20 and/or a phytase-containing granulate with an activity of at least 6,000 FTU/g.

24. A composition according to claim 23 which is an edible feed composition and/or an animal feed.

25. A composition according to claim 24 which comprises pellets of one or more feed substance(s) or ingredient(s) mixed with a granulate according to any of claims 15 to 20.

26. A composition according to any of claims 23 to 25 which is an animal feed, or a premix or precursor to an animal feed, preparable by a process according to claim 21 or 22.

27. A process for promoting the growth of an animal the process comprising feeding an animal with a diet that comprises a granulate as defined in any of claims 15 to 20 or a composition as defined in any of claims 23 to 25.

28. Use of a granulate as defined in any of claims 19 to 27 in, or as a component of, an animal feed or for use in a non-therapeutical animal diet.

29. Use of a composition comprising at least 15% (w/w) of an edible carbohydrate polymer as a carrier for a phytase to improve the pelleting stability of the phytase.

## Patentansprüche

1. Verfahren zur Herstellung einer eine Phytase enthaltenden wäßrigen Flüssigkeit, bei dem man:
(a) in einem wäßrigen Medium einen Mikroorganismus der Gattung *Aspergillus* oder *Trichoderma* mit einem heterologen Phytasegen unter der Kontrolle eines Glucoamylase-Promotors (für *Aspergillus*) oder Cellobiohydrolase-Promotors (für *Trichoderma*) unter Bedingungen kultiviert, die eine rekombinante Expression der Phytase erlauben, wobei das Medium als Nahrung für die Mikroorganismen eine assimilierbare Kohlenstoffquelle und eine aus Ammoniumionen bestehende assimilierbare Stickstoffquelle enthält;
(b) das wäßrige Medium zur Abtrennung der Mikroorganismen filtriert, wobei man ein wäßriges Filtrat erhält; und
(c) das Filtrat aus (b) einer Ultrafiltration unterwirft, wobei man eine wäßrige Flüssigkeit mit einer Phytasekonzentration von mindestens 14.000 FTU/g erhält.

2. Verfahren nach Anspruch 1, bei dem es sich bei dem Mikroorganismus um *Aspergillus niger, Aspergillus oryzae* oder *Trichoderma reesei* handelt.

3. Verfahren nach Anspruch 1 oder 2, bei dem der Mikroorganismus kein Glucoamylasegen (AG-Gen) aufweist oder exprimiert und/oder der Mikroorganismus multiple Kopien des Phytasegens aufweist und/oder die Phytase in dem Mikroorganismus mit einer Glucoamylase-Signalsequenz exprimiert wird.

4. Verfahren nach einem der vorhergehenden Ansprüche, bei dem die wäßrige Flüssigkeit weitgehend frei von Taka-Amylase ist und/oder die Kohlenstoff- und Stickstoffquellen in dem Medium vor dem Filtrieren in (b) von den Mirkoorganismen weitgehend vollständig verbraucht worden sind.

5. Verfahren nach einem der vorhergehenden Ansprüche, bei dem die wäßrige Flüssigkeit frei von den Kohlenstoff- und/oder Stickstoffquellen ist.

6. Verfahren nach einem der vorhergehenden Ansprüche, bei dem weder das wäßrige Filtrat noch die wäßrige Flüssigkeit einer Kristallisation und/oder einem Entfärbungsschritt unterworfen wird.

7. Verfahren nach einem der vorhergehenden Ansprüche, bei dem die resultierende wäßrige Flüssigkeit eine Phytaseaktivität von 18.000 FTU/g oder mehr aufweist.

8. Nach einem Verfahren nach einem der vorhergehenden Ansprüche herstellbare wäßrige Flüssigkeit, die Phytase in einer Konzentration von mindestens 14.000 FTU/g enthält.

9. Wäßrige Flüssigkeit nach Anspruch 8, die sich von einem Kulturmedium ableitet, in dem Phytase exprimiert wurde.

10. Verfahren zur Herstellung eines Phytase enthaltenden Granulats, das zur Verwendung in einem Tierfutter geeignet ist, bei dem man einen festen Täger, der mindestens 15 Gew.-% eines genießbaren Kohlenhydratpolymers enthält, und eine wäßrige Flüssigkeit nach Anspruch 8 oder 9 zu Phytase enthaltenden Granulatkörnern verarbeitet.

11. Verfahren nach Anspruch 10, bei dem man die wäßrige Flüssigkeit und den Träger vermischt und die erhaltene Mischung knetet und gegebenenfalls danach die Granulatkörner trocknet.

12. Verfahren nach Anspruch 10 oder 11, bei dem man:
a) die die Phytase enthaltende wäßrige Flüssigkeit mit dem festen Träger vermischt,
b) die in a) erhaltene Mischung mechanisch zu enzymhaltigen Granulatkörnern verarbeitet und
c) die in b) erhaltenen enzymhaltigen Granulatkörner trocknet.

13. Verfahren nach Anspruch 12, bei dem die Verarbeitung Extrudieren, Pelletieren, Granulieren unter hochscherenden Bedingungen, Expandieren, Wirbelschichtagglomeration oder eine Kombination davon umfaßt.

14. Nach einem Verfahren nach einem der vorhergehenden Ansprüche herstellbares Phytase enthaltendes Granulat.

15. Granulat, zumindest teilweise bestehend aus getrockneten Granulatkörnern aus einer Phytase und einem festen Träger, der mindestens 15 Gew.-% eines genießbaren Kohlenhydrats enthält.

16. Granulat nach Anspruch 15, bei dem die Granulatkörner mindestens ein zweiwertiges Kation und/oder ein oder mehrere hydrophobe, gelbildende oder wasserunlösliche Verbindungen enthalten.

17. Granulat nach Anspruch 16, bei dem es sich bei der hydrophoben, gelbildenden oder wasserunlöslichen Verbindung um eine derivatisierte Cellulose, Polyvinylalkohol (PVA) oder ein genießbares Öl handelt.

18. Granulat nach Anspruch 17, bei dem es sich bei der derivatisierten Cellulose um Hydroxypropylmethylcellulose, Carboxymethylcellulose oder Hydroxyethylcellulose und bei dem genießbaren Öl um Sojaöl oder Canolaöl handelt.

19. Granulat nach einem der Ansprüche 15 bis 18, das zusätzlich eine Endo-Xylanase und/oder β-Glucanase enthält und/oder bei dem der Träger Stärke enthält.

20. Granulat nach einem der Ansprüche 15 bis 19, bei dem es sich bei der Phytase nicht um eine hitzetolerante (thermostabile) Phytase und/oder um eine Pilzphytase, die gegebenenfalls von einer *Aspergillus-* oder *Trichoderma*-Art abgeleitet ist, handelt.

21. Verfahren zur Herstellung eines Tierfutters oder einer Vormischung oder Vorstufe für das Tierfutter, **dadurch gekennzeichnet, daß** man ein Phytase enthaltendes Granulat nach einem der Ansprüche 15 bis 20 mit einer oder mehreren Tierfuttersubstanzen oder einem oder mehreren Tierfutterbestandteilen vermischt.

22. Verfahren nach Anspruch 21, bei dem man die Mischung aus Futtersubstanz(en) und Zusammenetzung bzw. Granulat mit Wasserdampf behandelt, pelletiert und gegebenenfalls trocknet.

23. Zusammensetzung, die ein Granulat nach einem der Ansprüche 15 bis 20 und/oder ein Phytase enthaltendes Granulat mit einer Aktivität von mindestens 6.000 FTU/g enthält.

24. Zusammensetzung nach Anspruch 23, bei der es sich um eine genießbare Futterzusammensetzung und/oder ein Tierfutter handelt.

25. Zusammensetzung nach Anspruch 24, die Pellets aus einer oder mehreren Futtersubstanzen bzw. -bestandteilen in Abmischung mit einem Granulat nach einem der Ansprüche 15 bis 20 enthält.

26. Zusammensetzung nach einem der Ansprüche 23 bis 25, bei der es sich um ein Tierfutter oder eine Vormischung oder Vorstufe für ein Tierfutter handelt, die bzw. das nach einem Verfahren nach Anspruch 21 oder 22 hergestellt werden kann.

27. Verfahren zur Förderung des Wachstums eines Tiers, bei dem man an das Tier eine Nahrung verfüttert, die ein Granulat nach einem der Ansprüche 15 bis 20 oder eine Zusammensetzung nach einem der Ansprüche 23 bis 26 enthält.

28. Verwendung eines Granulats nach einem der Ansprüche 19 bis 27 in einem Tierfutter oder als Komponente eines Tierfutters oder zur Verwendung bei der nichttherapeutischen Tierernährung.

29. Verwendung einer mindestens 15 Gew.-% eines genießbaren Kohlenhydratpolymers als Träger für eine Phytase zur Verbesserung der Pelletierungsstabilität der Phytase.

## Revendications

1. Procédé pour la préparation d'un liquide aqueux comprenant une phytase, le procédé comprenant les étapes consistant à :
(a) cultiver dans un milieu aqueux un microorganisme du genre *Aspergillus* ou *Trichoderma* ayant un gène de phytase hétérologue sous la commande d'un promoteur de glucoamylase (pour *Aspergillus*) ou de cellobiohydrolase (pour *Trichoderma*), dans des conditions qui permettent l'expression recombinante de la phytase, le milieu comprenant, comme aliment pour le microorganisme, une source de carbone assimilable et une source d'azote assimilable consistant en ions ammonium ;
(b) filtrer le milieu aqueux pour éliminer les microorganismes afin d'obtenir un filtrat aqueux ; et
(c) soumettre le filtrat provenant de (b) à une ultrafiltration pour obtenir un liquide aqueux ayant une concentration en phytase d'au moins 14 000 FTU/g.

2. Procédé selon la revendication 1, dans lequel le microorganisme est *Aspergillus niger, Aspergillus oryzae* ou *Aspergillus reesei*.

3. Procédé selon la revendication 1 ou 2, dans lequel le microorganisme ne possède pas, ou n'exprime pas, un gène de glucoamylase (AG) et/ou le microorganisme possède des copies multiples du gène de phytase et/ou la phytase est exprimée dans le microorganisme avec une séquence signal de glucoamylase.

4. Procédé selon l'une quelconque des revendications précédentes, dans lequel le liquide aqueux est sensiblement exempt de taka-amylase et/ou dans lequel sensiblement la totalité des sources de carbone et d'azote dans le milieu ont été consommées par le microorganisme avant la filtration en (b).

5. Procédé selon l'une quelconque des revendications précédentes, dans lequel le liquide aqueux est exempt des sources de carbone et/ou d'azote.

6. Procédé selon l'une quelconque des revendications précédentes, dans lequel ni le filtrat aqueux ni le liquide aqueux ne sont soumis à une étape de cristallisation et/ou de décoloration.

7. Procédé selon l'une quelconque des revendications précédentes, dans lequel le liquide aqueux résultant a une activité phytasique de 18 000 FTU/g ou plus.

8. Liquide aqueux pouvant être préparé par un procédé selon l'une quelconque des revendications précédentes, comprenant une phytase à une concentration d'au moins 14 000 FTU/g.

9. Liquide aqueux selon la revendication 8, qui est dérivé d'un milieu de culture dans lequel la phytase a été exprimée.

10. Procédé pour la préparation d'un granulat contenant de la phytase, utilisable dans un aliment pour animaux, le procédé comprenant la transformation d'un support solide comprenant au moins 15 % (en poids) d'un polymère glucidique comestible et d'un liquide aqueux selon la revendication 8 ou 9 pour produire des granulés contenant de la phytase.

11. Procédé selon la revendication 10, comprenant le mélange du liquide aqueux et du support, et le malaxage du mélange résultant, puis facultativement le séchage des granulés.

12. Procédé selon la revendication 10 ou 11, dans lequel le procédé comprenant :
a) le mélange du liquide aqueux contenant la phytase avec le support solide ;
b) la transformation mécanique du mélange obtenu en a) pour obtenir des granulés contenant l'enzyme ; et
c) le séchage des granulés contenant l' enzyme obtenus en b).

13. Procédé selon la revendication 12, dans lequel la transformation comprend une extrusion, une pelletisation, une granulation à fort cisaillement, une expansion, une agglomération en lit fluide ou une combinaison d'entre elles.

14. Granulat contenant de la phytase pouvant être préparé par un procédé selon l'une quelconque des revendications 10 à 13.

15. Granulat comprenant des granulés séchés formés à partir d'une phytase et d'un support solide qui comprend au moins 15 % (en poids) d'un polymère glucidique comestible.

16. Granulat selon la revendication 15, dans lequel les granulés comprennent au moins un cation divalent et/ou un ou plusieurs composés hydrophobes, formateurs de gel ou insolubles dans l'eau.

17. Granulat selon la revendication 16, dans lequel le composé hydrophobe, formateur de gel ou insoluble dans l'eau comprend une cellulose modifiée, de l'alcool polyvinylique (PVA) ou une huile comestible.

18. Granulat selon la revendication 17, dans lequel la cellulose modifiée est l'hydroxypropylméthylcellulose, la carboxyméthylcellulose ou l'hydroxyéthylcellulose et/ou l'huile comestible est l'huile de soja ou l'huile de canola.

19. Granulat selon l'une quelconque des revendications 15 à 18, qui comprend de plus une endoxylanase et/ou une β-glucanase et/ou dans lequel le support comprend de l'amidon.

20. Granulat selon l'une quelconque des revendications 15 à 19, dans lequel la phytase est autre qu'une phytase tolérante à la chaleur (thermostable) et/ou une phytase fongique, ladite phytase fongique dérivant facultativement d'une espèce de *Aspergillus* ou *Trichoderma.*

21. Procédé pour la préparation d'un aliment pour animaux, d'un prémélange ou d'un précurseur de l'aliment pour animaux, le procédé comprenant le mélange d'un granulat contenant de la phytase selon l'une quelconque des revendications 15 à 20 avec un ou plusieurs ingrédients ou substances alimentaires pour animaux.

22. Procédé selon la revendication 21, dans lequel le mélange d'une ou plusieurs substances alimentaires et la composition ou le granulat est traité à la vapeur d'eau, pelletisé et facultativement séché.

23. Composition comprenant un granulat selon l'une quelconque des revendications 15 à 20 et/ou un granulat contenant de la phytase ayant une activité d'au moins 6000 FTU/g.

24. Composition selon la revendication 23, qui est une composition d'aliment comestible et/ou un aliment pour animaux.

25. Composition selon la revendication 24, qui comprend des pellets d'un ou plusieurs ingrédients ou substances alimentaires mélangés avec un granulat selon l'une quelconque des revendications 15 à 20.

26. Composition selon l'une quelconque des revendications 23 à 25, qui est un aliment pour animaux, ou un prémélange ou précurseur d'un aliment pour animaux, pouvant être préparé par un procédé selon la revendication 21 ou 22.

27. Procédé pour activer la croissance d'un animal, le procédé consistant à nourrir un animal avec une alimentation qui comprend un granulat tel que défini dans l'une quelconque des revendications 15 à 20 ou une composition telle que définie dans l'une quelconque des revendications 23 à 26.

28. Utilisation d'un granulat tel que défini dans l'une quelconque des revendications 19 à 27 dans, ou comme composant de, un aliment pour animaux ou son utilisation dans une alimentation non thérapeutique d'animaux.

29. Utilisation d'une composition comprenant au moins 15 % (en poids) d'un polymère glucidique comestible comme support pour une phytase afin d'améliorer la stabilité de la phytase à la pelletisation.
